# EUROPEAN PATENT SPECIFICATION

(11) **EP 2 223 774 B1**
(45) Date of publication and mention of the grant of the patent: **08.06.2011**
(21) Application number: 09153876.9
(22) Date of filing: 27.02.2009
(51) Int. Cl.: B24B 17/02, B24B 47/00, D06C 29/00, G01N 3/56

(54) **Surface abrader and apparatus for testing surface abrasion**
Oberflächenschleifgerät und Vorrichtung zur Prüfung des Oberflächenschliffs
Abrasif de surface et appareil pour tester l'abrasion de surface

(43) Date of publication of application: 01.09.2010
(73) Proprietor: RESEARCH IN MOTION LIMITED, Waterloo, Ontario N2L 3W8 (CA)
(72) Inventor: Pemberton-Pigott, Nigel P., Waterloo, Ontario N2L 3L3 (CA)
(74) Representative: MERH-IP Matias Erny Reichl Hoffmann

(56) References cited:
- EP-A- 1 316 791
- EP-A- 1 630 538
- CN-Y- 2 893 691
- US-A- 3 107 457

## Description

### Field

This technology relates to a surface abrader and apparatus for testing surface abrasion.

### Background

Known surface abraders typically move in a circle or in a straight line back and forth during operation. Such conventional devices typically have a rigid tip that points downwardly in the vertical direction. One known surface abrader is the Martindale Abrasion Tester, which is primarily designed to determine the abrasion resistance of textile fabrics. Abrasion resistance is measured by subjecting the specimen to a rubbing motion in the form of a geometric figure. Resistance to abrasion is evaluated by various means, including comparison to visual aids in the form of photographs or actual samples.

Another known surface abrader is the Taber Linear Abrader. This device is becoming more recognized for conducting physical testing of decorated plastics and other contoured products. It uses a horizontal arm that reciprocates in a linear motion and includes a "free-floating" test system attached at one end of the arm. As the arm cycles back and forth, a spline shaft raises or lowers the arm as the test attachment follows the contours of the specimen being tested. This device can utilize interchangeable abrader tips.

Both of these abraders abrade in a straight line or a circle. Although some conventional abraders disclose random motion, the motion is controlled by a computer, which requires expensive programming and software.

An abrasion tester according to the preamble of claim 1 is disclosed in document EP 1 316 791.

### Brief Description of the Drawing Figures

Fig. 1 is a top view of an example surface abrading device;
Fig. 2 is a depiction of various abrading patterns that are possible with the abrading device of Fig. 1;
Fig. 3 depicts a prior art heim joint, which is a component used in the example abrading device;
Fig. 4 is a top view of another example surface abrading device;
Fig. 5 is a top view of yet another example surface abrading device;
Fig. 6 is a top view of a further example surface abrading device;
Fig. 7 is a top view of another example surface abrading device;
Fig. 8 is a top view of yet another example surface abrading device;
Fig. 9 is a top view of a further example surface abrading device;
Fig. 10 is a top view of another example surface abrading device;
Fig. 11 is a top view of yet another example surface abrading device;
Fig. 12 is a top view of a further example surface abrading device;
Fig. 13 is a top view of another example surface abrading device;
Fig. 14 is a top view of yet another example surface abrading device;
Fig. 15 is a top view of a further example surface abrading device;
Fig. 16 is a front view of an example surface abrading device, similar to that shown in Fig. 7;
Fig. 17 is a side view of an example surface abrading device with a different abrasion tool holder that causes the abrader tip to push harder down onto the working plane when pulling back;
Fig. 18 is a side view of an example surface abrading device with a different abrasion tool holder that causes the abrader tip to push harder down onto the working plane when pushing forward;
Fig. 19 is a right side view showing the yaw and pitch of an abrader tip utilized with the example surface abrading device of Fig. 9;
Fig. 20 is an opposite left side view showing the stroke of an abrader tip utilized with the example surface abrading device of Fig. 9; and
Fig. 21 is a diagrammatic representation of the roll of a pointer utilized with the example surface abrading device of Fig. 12.

### Detailed Description

The example surface abrader 10 can abrade in multiple shapes, including rectangles, ovals, circles, and diamond shapes, among other shapes. The example abrader 10 can also move in two horizontal axes. The example device 10 provides an abrader tip 12 motion that moves like a human finger across a surface, instead of a rigid tip that is always pointing downwardly, as with conventional devices. Because the example surface abrader 10 can provide more human-like motions, it can be used to more closely mimic real life operation and degradation of the keys and surfaces of, among other devices, mobile communication devices. The example device 10 accomplishes its movement without the need for expensive software or computer programming.

The example device 10 can be used to test the touch screen of a mobile device, or any surface that needs to be tested for large area abrasion, such as the outer surface of a battery door of a mobile device. Other devices, not limited to mobile devices, may also draw utility from the examples described herein. The example surface abrader 10 has the capability to test not only an area, but a straight line. As a result, it can be used to test side grips of mobile devices, or anything where a straight line abrasion would be required, such as keypads. In addition, the example surface abrader 10 has the ability to test things with a finger-tip-like device that tilts and swivels around the surface being tested. These finger-like or human-like movements allow the device to more accurately mimic the effect of dirt and oils being wiped into gaps of a keypad, for example. An abrasion tool 12 in the form of a ball end (not shown) could roll around the surface of the device 10 and keypad and press on the various keys with a lubricant in order to "dirty" the surface. Thus, the device 10 can be used to mimic real world operation.

The human-like movements of the example device 10 are advantageous because they allow testing on keys and keypads. Running a finger-like pointer that swivels and rolls and that is coated with foreign substances over some keys will jam some of the foreign substance in between the cracks and crevices of the keys. A simple straight line abrader, as with conventional testing devices, would not be able to do this. The ability to mimic real world operation allows the user to more effectively test a surface and consider any potential degradation of keys or surfaces. The example device 10 can be used to determine if the natural motion of a user's hands will pull up components or displace them sideways or down. The example device 10 is an effective way for testing how dirt accumulates in keypad areas, jog balls, and any mating parts of the mobile device that have gaps or do not match well together.

The example surface abrader 10 is able to abrade an entire surface, such as an entire touch screen, instead of only in a straight line, as with conventional testing devices. Conventional surface abraders often used a larger surface area tool to abrade a surface. This was akin to a big block. The big block was used to cover an entire surface area, with the block moving back and forth to perform the abrasion. Unlike conventional surface abraders, the example device 10 allows testing with a finger-tip type device instead of with a big block that covers an entire surface. Abrasion using big blocks is different in terms of precision compared with the use of a small swiveling finger-tip to cover the same area. The use of a large surface area tool to abrade in a straight line also limits the abrasion testing to a flat surface only. With the present examples, different types of pointers or abrasion tools 12 may be used to accomplish different results. The example surface abrader 10 is capable of abrading an entire surface, whether or not it is flat, such as a keypad, and can maneuver over small hills into valleys. The area being abraded can also be easily changed by means of a simple adjustment to a few pins, which are positioned on pulleys P1, P2, P3, P4, flanges F1, F2, F3, F4, F5, F6 or other parts of the device BE I, BE2 (described in further detail below), or by adjusting the speed of the variable speed motors M1, M2. The example device 10 accomplishes this without the need for specially controlled motors or software.

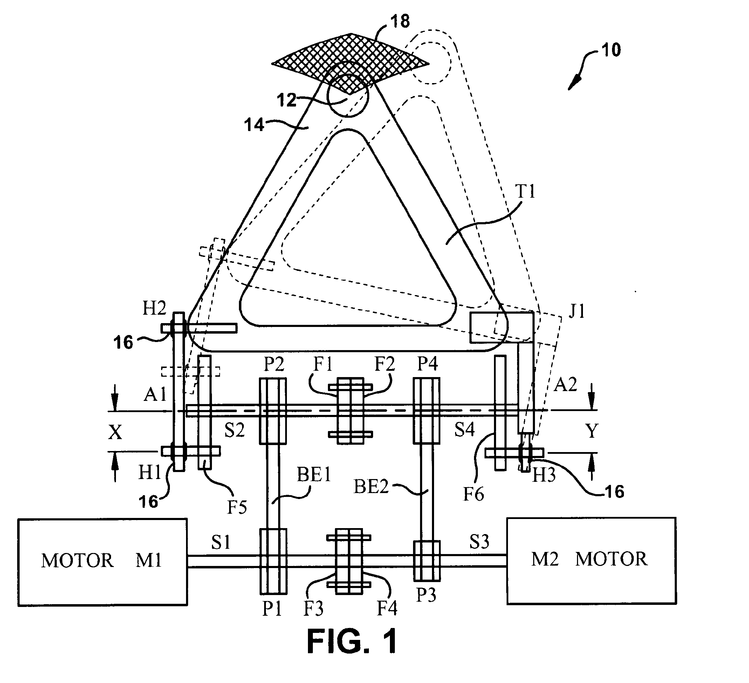

Referring to the drawings, Fig. 1 depicts an example surface abrader and apparatus for testing surface abrasion 10, also known as an area tester abrasion device. The device 10 is designed to closely follow a surface and includes a series of pulleys P1, P2, P3, P4 positioned on shafts S1, S2, S3, S4 and variously coupled to either or both motors M1 or M2. The shafts include a distal or first shaft, which comprises a first shaft portion S1 and a second shaft portion S3. The first and second shaft portions S1, S3 may be referred to herein as the distal shaft portions, distal shafts, or simply shafts. The shafts S1, S3 are spaced from a proximal shaft, which comprises a first shaft portion S2 and a second shaft portion S4. The first and second shaft portions S2, S4 may be referred to herein as the proximal shaft portions, proximal shafts, or simply shafts. Distal shaft portions S1, S3 may be axially aligned and proximal shaft portions S2, S4 may be axially aligned, although this is not required in all examples. Shafts S1, S3 are parallel to shafts S2, S4, although there may be examples where the distal shafts are not parallel to the proximal shafts.

The example abrasion device 10 has a first motor M1 and a second motor M2. One or both motors M1, M2 can be used to drive first and second arms A1, A2, which are coupled to the frame T1. An abrader tool 12 is coupled to the frame T1. In order to move the frame T1 and abrader tool 12, the device 10 includes a plurality of variable speed and diameter pulleys P1, P2, P3, P4 that are positioned on shafts S1, S2, S3, S4. In the example shown, variable speed pulleys are pulleys P1, P3 and variable diameter pulleys are P2, P4, although pulleys P1, P3 could be of variable diameter and pulleys P2, P4 could be of variable speed. The pulleys are coupled to one another with belts. A belt BE1 couples pulley P1 to pulley P2. A belt BE2 couples pulley P3 to pulley P4. Four shafts are shown, including shaft S1 that is directly connected to motor M1, a shaft S3 that is directly connected to motor M2, a shaft S2 that is coupled to shaft S1 via belt BE1 and to arm A1, and a shaft S4 that is coupled to shaft S3 via belt BE2 and to arm A2.

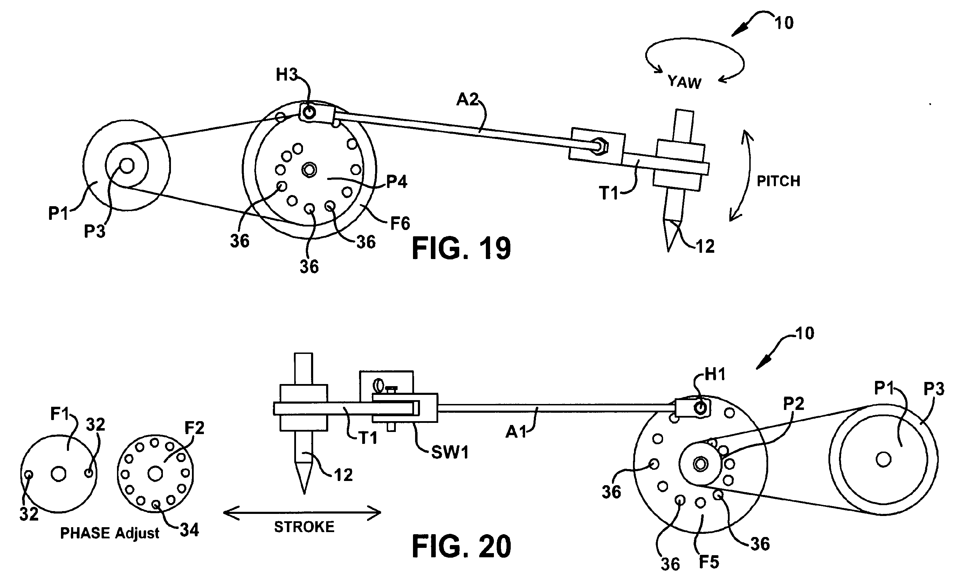

The flanges F3, F4 may couple the shafts together such that shafts S 1, S3 rotate together at the same speed (in which case one of the motors M1, M2 would be quieted), or may be disconnected, such that shafts S1, S3 rotate separately from one another at different speeds, as governed by the associated motors M1, M2. Shafts S2, S4 may be coupled via flanges F1 and F2, or may operate separately from one another. Flanges F1, F2, F3, F4, F5, F6 include pins or other connectors and openings for receiving the pins or connectors that allow the arms A1, A2 to be either in or out of phase with one another as flanges F5 and F6 rotate, as shown more clearly in Fig. 20, and as described in greater detail below. The flanges F1, F2 may couple shafts S2, S4 together so that they rotate together at the same speed, or may be disconnected so that shafts S2, S4 may rotate at different speeds. Coupling flanges F1 and F2 allow shafts S2, S4 to rotate along a common axis, while shafts S1 and S3 are freed from this constraint. In this case, motors M1 and M2 would both be required. Coupling flanges F3 and F4 would allow shafts S1 and S3 to rotate along a common axis, while shafts S2 and S4 would be freed from this constraint. In this case either motor M1 or M2 would be required. Alternatively, flanges F1, F2, F3, and F4 can be left uncoupled and this would then allow shafts S1, S2, S3, and S4 to all rotate along non-collinear axes. In this case, either motors M1 or M2 or both can be utilized.

Flanges F1, F2, mounted respectively on shafts S2, S4, and flanges F3, F4 mounted respectively on shafts S1, S3 each have at least one hole to permit their being connected to each other: F1 to F2 or F3 to F4. These holes are shown best in Fig. 20, where both flange F2 and flange F2 are disc shaped. Flange F1 includes two holes 32 and flange F2 includes numerous holes 34 that are spaced around the periphery of flange F2. As will be discussed in greater detail below, the phase of the device 10 may be adjusted based upon how flange F2 is rotated relative to flange F1. One or more pins may be used to couple flange F1 to flange F2, and/or flange F3 to flange F4. Other connecting fasteners may alternatively be utilized.

Flanges F5, F6 are mounted respectively on shafts S2, S4 and are coupled to arms A1, A2, respectively. The arms A1, A2 are coupled to frame T1, which is coupled to the abrader tip or pointer 12. Pointer 12 is used to contact a surface to be tested or abraded. Arms A1, A2 are coupled to flanges F5, F6 via joints H1, H3. Frame T1 is shown coupled to arm A1 via joint H2.

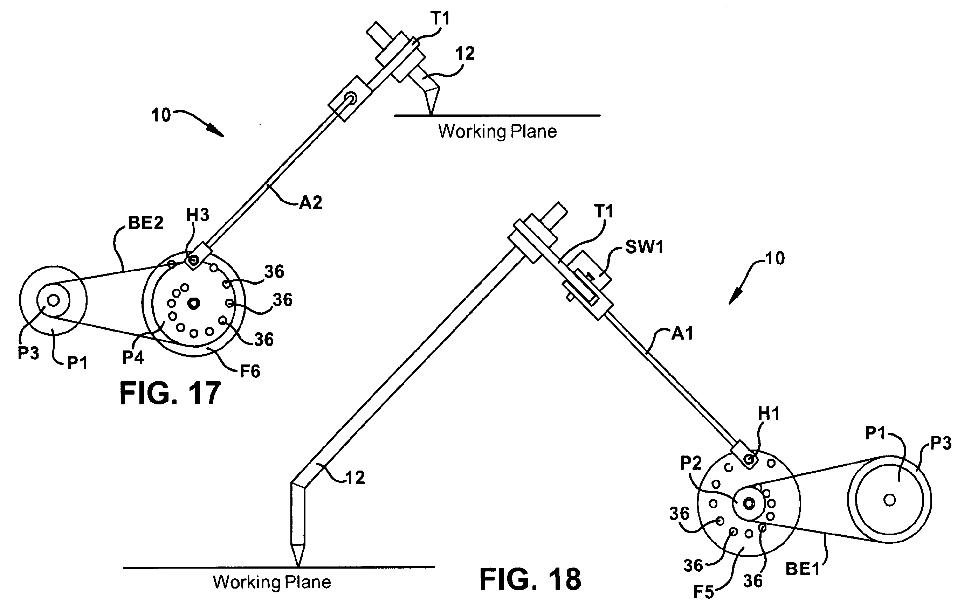

Flanges F5, F6 are disc-shaped and each have a plurality of holes that are provided through the disc and spaced from one another in a spiral shape on the face of each disc. This is shown best in Figs. 17-20. These holes 36 are referred to as arm offset holes and each hole is used to eccentrically mount a precision articulating joint, such as heim joints H1, H3 described in greater detail below. These holes also allow the arms A1, A2 to be offset from the center of flanges F5, F6 by distances x and y, respectively, as shown in Fig. 1. Distances X and Y are determined by which hole 36 is chosen for the location of the respective arm. Flanges F5, F6 allow the arms to be offset relative to one another, which allows for greater flexibility in terms of abrasion patterns that are possible with the example device.

Shafts S1, S3 may be joined together axially by at least one pin through rotating flanges F3, F4. Arms A1, A2 are mounted on joints H1, H3, respectively, which are in turn mounted on rotating flanges F5, F6 respectively. Depending upon how the arms are coupled to flanges F5, F6 and how the flanges F1, F2, F3, F4 are coupled to one another, the device 10 is induced to provide a back and forth motion of frame T1. Both of the joints H1, H3 move axially as flanges F5, F6 rotate about shafts S2, S4 utilizing motors M1 and/or M2. As is evident, many different configurations are possible for the present device and any number of different abrasion patterns are possible based upon which holes 34, 36 are selected, which pulleys are belted, which motors are utilized, and which flanges are connected to one another, among other choices. A variety of different configurations are discussed below.

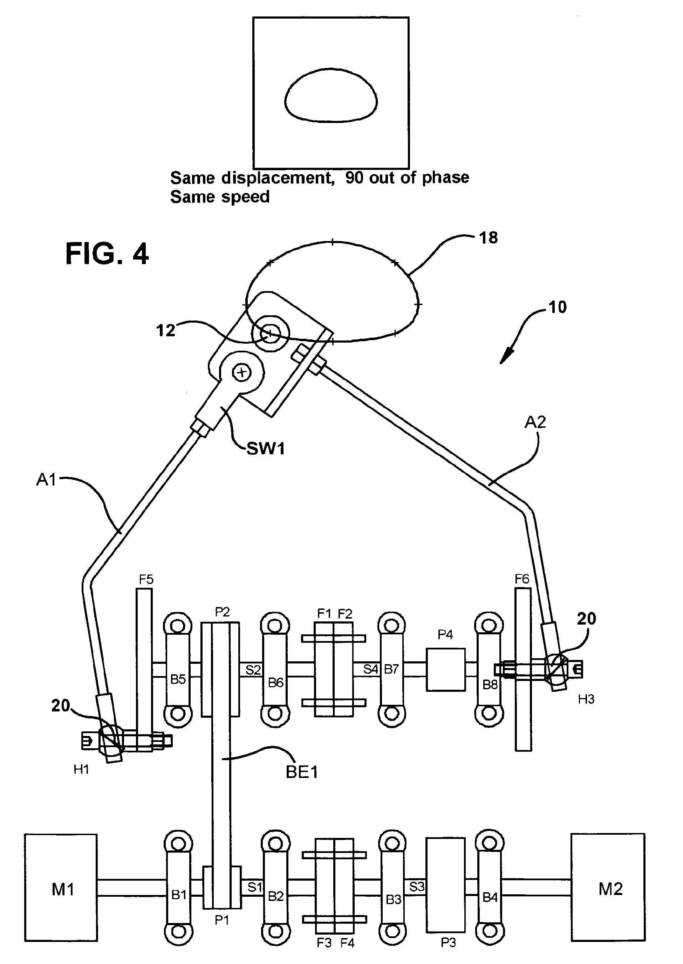

In contrast to Fig. 1, where the abrader tip 12 is coupled to a frame T1, in Figs. 4-15, arm A1 is shown coupled to the abrader tip 12 via a swivel joint SW1. Arm A2 is fixed to the abrader tip 12 The swivel joint SW1 allows the abrader tip 12 to have free movement. Without the swivel joint SW1 or some similar joint that allows for free movement of the abrader tip 12, the abrader tip would likely break.

The two arms A1, A2 ca-n operate in phase with one another or out of phase with one another. The amount of phase difference and the speed of the arms relative to one another helps to shape the abrasion pattern provided by the device 10. Figs. 4-15 show a variety of different abrasion patterns that are provided by different phase shifts and speeds of operation. The phase of the device may be determined by flanges F1, F2 or flanges F3, F4 along with pulleys P1, P2, P3, P4.

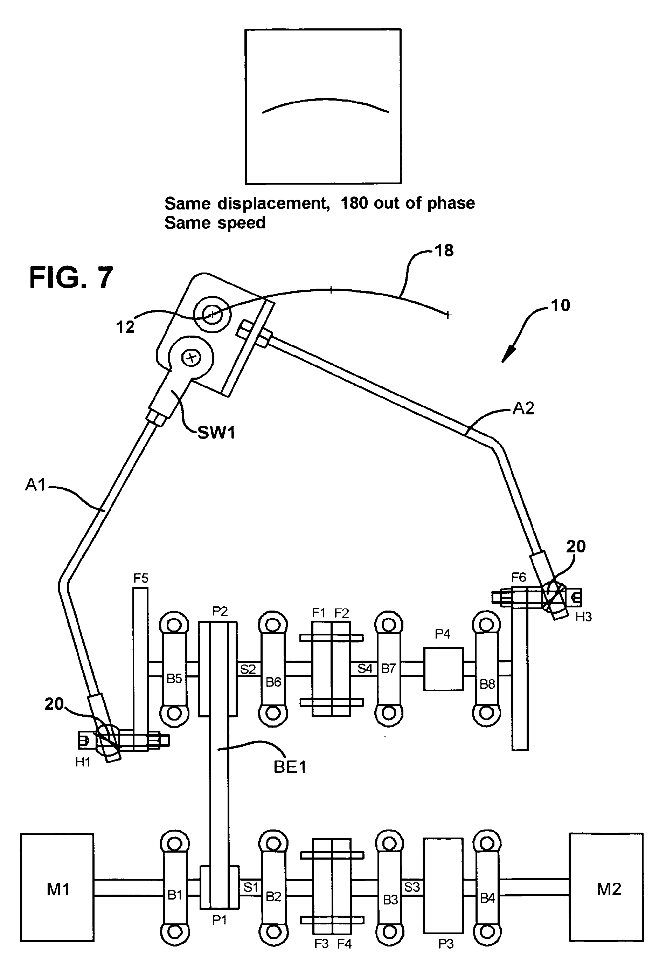

With respect to phase shifts created utilizing flanges F1, F2, as previously discussed, flange F2 has a plurality of holes 34 spaced around the periphery thereof. In order to understand phase, consider flanges F5, F6. When the flanges F1, F2 are in phase with one another, a fixed point on flange F5 will always align with a fixed point on flange F6. When flanges F1, F2 are out of phase with one another, such as by rotating flange F2 90 degrees relative to flange F1, the fixed point on flange F5 will be rotated 90 degrees relative to the same fixed point on Flange F6. In this example, the fixed point on flange F5 will always be 90 degrees different from the fixed point on flange F6. Examples of configurations with phase shifts are shown in Figs. 4 and 7.

It is also possible to have a device 10 where the phase between flange F5 and flange F6 varies constantly. In this example, which is not shown, flange F5 may be rotationally fixed while flange F6 rotates. Here the fixed point on flange F5 will always remain at the same location while the fixed point F6 on flange F6 constantly rotates. In this example, flange F6 will have phase shift from 0 to 360 degrees. All the while, flanges F5, F6 may remain axially aligned, but the arms A1, A2 may be out of phase with one another, as known by those of skill in the art. The speed of flanges F5, F6 may vary depending upon whether or not flanges F1, F2 are connected to one another. Speed of operation is different from the phase of the arms A1, A2 relative to one another.

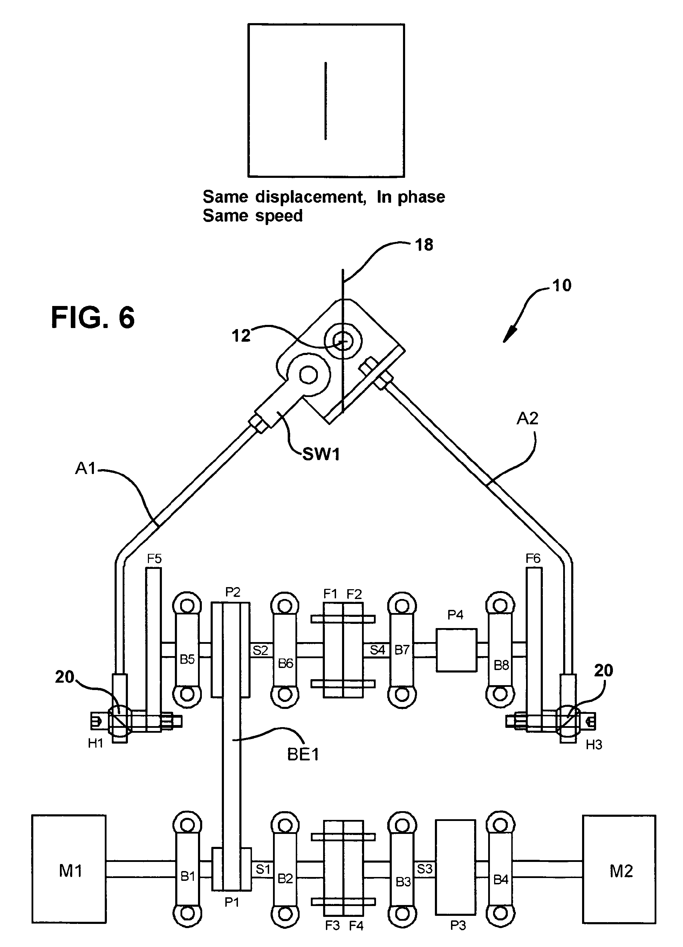

Different phase shifts are shown in the examples of Figs. 4-15. In Fig. 4, arm A2 is shifted 90 degrees relative to arm A1. This is evident from the location of joint H1, which is positioned at the bottom of flange F5 while joint H3, and thus arm A2, is rotated 90 degrees on flange F6 relative to the location of joint H1 on flange F5. Fig. 6 shows an example where arms A1, A2 are in phase with one another. This is evident from the location of joint H1, which is positioned at the bottom of flange F5 compared to the location of joint H3, which is positioned at the bottom of flange F6. Since flanges F5, F6 are operating at the same speed, arms A1, A2 will remain in phase with one another. Fig. 7 shows arm A1 being out of phase with arm A2 by 180 degrees. This is evident from the location of joint H1, which is positioned at the bottom of flange F5 while joint H3 is positioned at the top of flange F6. Since flanges F5, F6 are operating at the same speed, arms A1, A2 will remain 180 degrees out of phase.

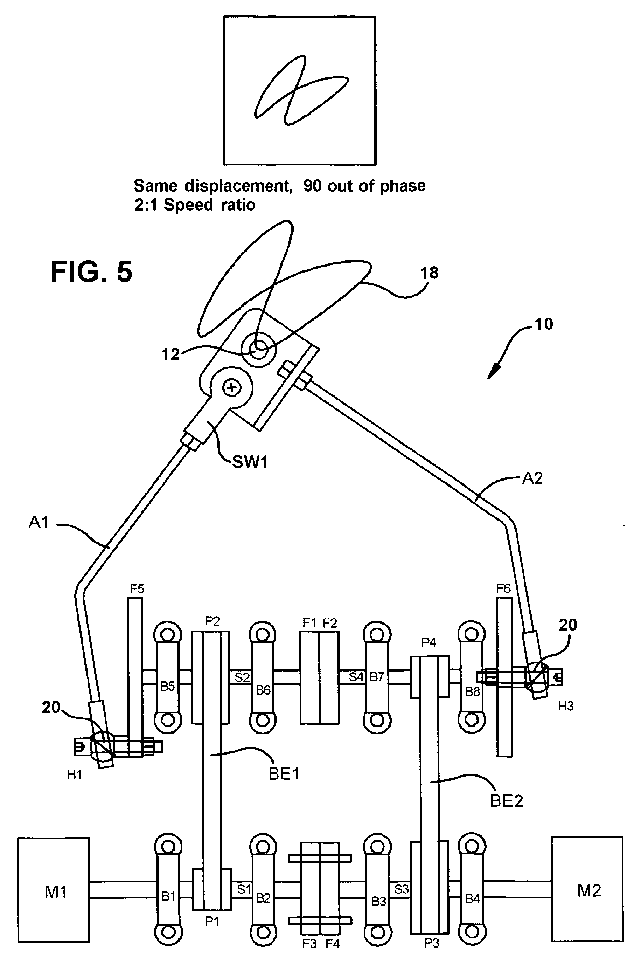

It is also possible to vary the phase of arm A1 relative to arm A2 when flanges F1, F2 are not connected to one another. This may be accomplished by coupling flanges F3, F4 together while flanges F1, F2 are not connected to one another. An example of this is shown in Fig. 5. In this example, at a starting point as shown, joint H1 is out of phase 90 degrees relative to joint H3. Pulleys P1, P2 are coupled together with belt BE1 and pulleys P3, P4 are coupled together with belt BE2. Pulley P1 is coupled to motor M1 and pulley P3 is coupled to motor M2. Motors M1 and M2 are operating at different speeds. In this configuration, the phase of arm A1 relative to arm A2 varies from 0 to 360 degrees. If pulleys P1, P2, P3, P4 were configured the same way such that shafts S2 and S4 rotated at the same speed, it would be possible to keep the same phase shift of 90 degrees (or any other angle), although this is not shown in Fig. 5. Thus, while the description in Fig. 5 states that the arms are 90 degrees out of phase, this phase shift will vary as the device moves.

The speed of the arms A1, A2 may be varied relative to one another. This may be accomplished by connecting flanges F3, F4 axially, while allowing flanges F1, F2 to remain uncoupled. Pulleys are selected that have different sizes such that flanges F5, F6 rotate at different speeds while utilizing a single motor, either M1 or M2. Examples of this are shown in Figs. 5 and 8-15. Shafts S2, S4 could be axially aligned with one another in this example, although that is not critical.

Flanges F1, F2, F3, F4 may be disconnected from one another and motors M 1 and M2 may control the movement of the flanges either mechanically or electronically so as to produce any combination of in phase or out of phase rotation of joints H1, H3 and arms A1, A2. Any differential speed of rotation of shafts S2 and S4 may be produced by this combination to provide a wide range of motion for frame T1 and the abrasion tool 12.

During operation, the arms move frame T1 in a reciprocal motion while holding an abrasion tool 12. The abrasion tool 12 is attached to the top end 14 of frame T1, which is shown in Fig. 1 as being triangular in shape, although other shapes may be utilized. Joints H1, H2, H3 are positioned between the frame T1 and the pulleys P1, P2, P3, P4 in order to allow the frame T1 and abrasion tool 12 to move in a reciprocating motion that is not limited to the dimensions of the triangle-shaped frame T1. The abrasion tool 12 shown is a pointer and moves in a pattern 18 when positioned against a surface to be abraded or tested, such as the surface of a mobile device. Other types of abrasion tools may also be utilized.

In Fig. 1, flange F5 is shown coupled between shaft S2 and arm A1. Flange F5 is coupled to arm A1 at a lower end of the arm A1 via a joint, which in this example is a heim joint H1. The lower end of arm A1 is coupled a distance x from the axis of rotation of shaft S2. A heim joint H2 is positioned at the upper end of arm A I and couples arm A1 to the frame T1. A flange F6 is shown coupled between shaft S4 and arm A2. Flange F4 is coupled to arm A2 at a lower end of the arm A2 via a heim joint H3. Heim joint H3 is spaced a distance y from the axis of rotation of shaft S4. An upper end of arm A2 is coupled to the frame T1 via a welded joint J1 such that arm A2 is fixedly connected to the frame T1. Joint J1 is a stationary joint. Other types of joints may be used, if desired.

During operation, variable speed pulley P1 turns variable diameter pulley P2 on shaft S2. Variable speed pulley P3 turns variable diameter pulley P4 on shaft S4. Shaft S2 turns arm A1 and shaft S4 turns arm A2. Arms A1, A2 reciprocate either in or out of phase with each other depending on the connections and pulleys as described above, which moves the frame T1. Joints H1, H2, H3, which are described in greater detail below, allow the arms A1, A2 to reciprocate without being restricted by the dimensions of the frame T1. The abrader tip 12 could be coupled to the frame T1 at other points. In addition, while the frame T1 is shown as being triangular, other shapes could be used for the frame.

Figs. 4-15 show how bearings B1, B2, B3, B4, B5, B6, B7, B8 may also be interspersed between the various parts. Bearings, such as pillow block bearings, may also be used in the example shown in Fig. 1, although not shown. Bearings B1, B2, B3, B4, B5, B6, B7, B8 may be positioned between each moving part, such as on either side of each pulley P1, P2, P3, P4 and adjacent to each motor M1, M2 and flanges F1, F2, F3, F4, F5, F6.

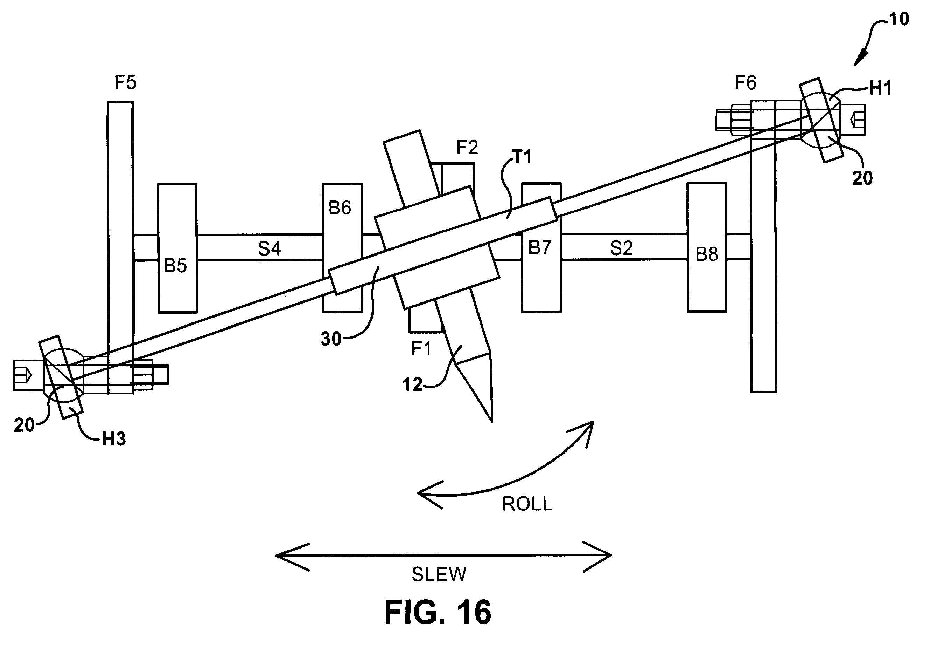

During operation, the abrader tip 12 moves in a pattern 18 represented by the shaded area at the top end 14 of the frame T1, as shown in Fig. 1. The range of motion of the frame T1 in two dimensions, known as slew, is shown in Fig. 1. Slew is movement from side to side. Fig. 16 is a front view of a device 10 similar to that shown in Fig. 1, showing how the movement of the device 10 has both a roll and slew. In Fig. 16, slew is shown as movement from side to side (left and right) while roll is shown as the rotating movement of the pointer 12. The roll movement is similar to the movement of a pendulum. Side views Fig. 19 and 20 show various views of the device 10 and depicts the yaw (axial rotation of the tip), pitch (front to back axial tilt), and stroke (back and forth movement) of the device 10. These various movements work together to produce an abrasion pattern 18, such as that shown in the figures.

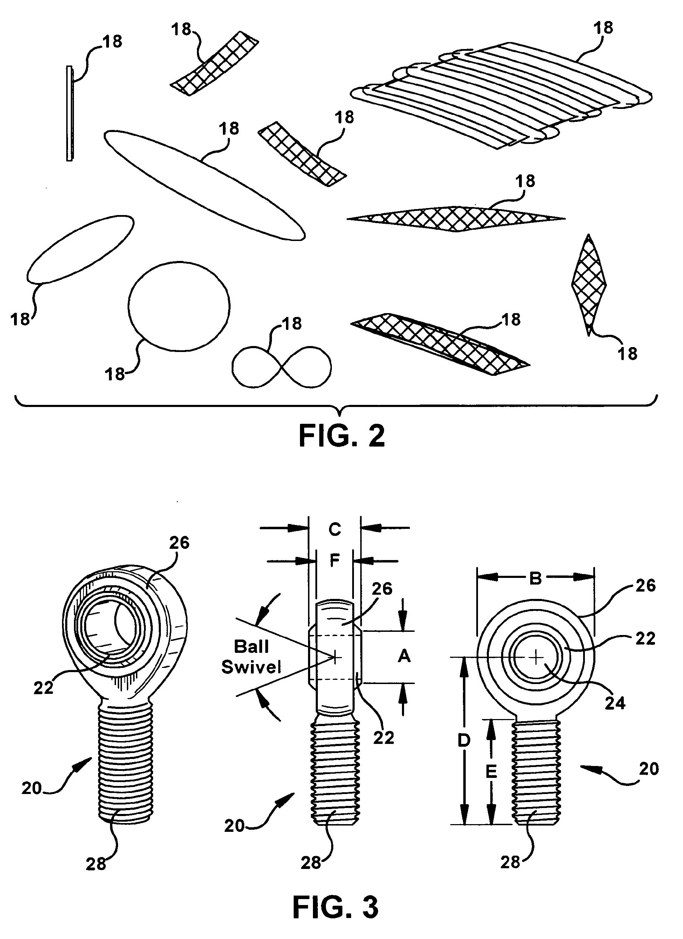

Other abrasion patterns 18 are shown in Fig. 2. These various patterns include circular, oval, figure eight, straight line, diamond shaped, and rectangular. The number of patterns and shapes possible with the example device 10 is almost limitless. Some of the patterns are linear in nature, such as a straight line, an arc, a circle, an oval, or a trapezoid. Other patterns cover a complete surface area, such as the diamond pattern shown in Fig. 2. It is also possible to obtain random motion of the abrader tip 12.

Referring to Fig. 3, a heim joint 20 (H1, H2, H3), also known as a rod end bearing, is shown. A heim joint is a type of joint that may be used with the present examples and is a mechanical articulating joint that is typically used on the ends of control rods, steering links, tie rods, or anywhere a precision articulating joint is required. The heim joint is useful in the present examples because it provides an inexpensive, simple, strong, multi-axis joint that provides a wide range of motion. There are different types of heim joints that may be utilized, such as a rotating or sliding heim joint. Other types of joints, such as universal joints or other joints, may alternatively be used. A heim joint H1, H2, H3 includes a ball swivel 22 with an opening 24 through which a bolt or other attaching hardware (not shown) may pass. The ball swivel 22 is pressed into a circular casing 26 that has a threaded shaft 28 attached. The threaded portion 28 may be either male or female. As shown in Fig. 3, the heim joint 20 has a male threaded portion with a casing 26 having a width B and a thickness F, a ball swivel 22 having a thickness C, an opening 24 for attaching hardware having a diameter A, and a threaded shaft 28 having a length E. The center of the opening 24 is spaced D from the bottom of the threaded shaft 28.

Using the example device, it is possible to obtain abrasion coverage over a complete surface area. In order to accomplish this, the device 10 can be configured so that shaft S2 and shaft S4 are not connected together via flanges F 1 and F2. In addition pulleys P1, P2, P3, P4 may not have the same diameters--and arms A1, A2 may not have the same lengths. Different degrees of area coverage can be obtained by increasing or decreasing the differences in diameter between the pulleys P1, P2, P3, P4 and between the offset lengths x, y and various lengths of arms A1, A2.

It is also possible, using the device, to obtain abrasion patterns 18 in an arc, circle, oval, or trapezoid. In order to abrade in an arc pattern, flanges F3, F4 are connected and set to 180 degrees phase difference while flanges F2 and F2 are disconnected. Alternatively, an arc pattern may be created when the flanges F1 and F2 are connected 180 degrees out of phase and shafts S2 and S4 rotate at the same speed. In order to abrade in a generally circular or oval pattern, flange F2 is coupled to flange F1 such that there is a 90 degree phase difference between the joints H1, H3. In order to abrade in a trapezoid pattern flanges F5 and F6 should be rotating at different speeds.

It is further possible to obtain random motion of the abrader tip 12. In order to obtain random motion, the second motor M2 is connected to the fourth shaft S4 and is set at a speed that is not a real number fraction of the speed of the first motor M1. The first arm A1 and the second arm A2 have different offset lengths, x and y, that are not a real number fraction of one another.

The size, orientation, and area of coverage of all the abrading patterns and shapes 18 can be adjusted by rotating the angle of phase shift between flanges F1, F2, F3, F4, by changing the diameters of the variable diameter pulleys P1, P2, P3, P4, by adjusting the length of the offsets x and y of arms A1 and A2, by adjusting the position of the Heim joints H1, H3 respectively on Flanges F5, F6, and by adjusting the speeds of motors M 1, M2.

Various examples of patterns 18 that may be created by configuring the device accordingly are shown in Figs. 4-15. The devices 10 shown in Figs. 4 - 15 are somewhat different from the device shown in Fig. 1. In particular, the triangle-shaped frame is replaced by two longer arms, only two heim joints are used and one swivel joint SW1, and the abrasion tool is coupled to the frame T1. The first and second arms A1, A2 create a frame by their size and shape. The abrasion tool 12 may be a pointer, such as shown in Figs. 16-20. When the abrasion tool is a pointer, the frame T1 is also a pointer holder.

Fig. 4 depicts a circular motion for the abrading tool 12. In this example, a single motor M1 or M2 drives the device via belt BE1. As shown, bearings B1, B2, B3, B4 are positioned between motor M1 and pulley P1, pulley P1 and flange F3, flange F4 and pulley P3, and pulley P3 and motor M2, respectively. Bearings B5, B6, B7, B8 are also positioned between flange F5 and pulley P2, pulley P2 and flange F1, flange F2 and pulley P4, and pulley P4 and flange F6, respectively. The example shown in Fig. 4 is an example device 10 where the arms A1, A2 have the same displacement (x=y) and are 90 degrees out of phase, but operating at the same speed.

Fig. 5 depicts an example device 10 where the arms A1, A2 have the same displacement (x=y), are operating 90 degrees out of phase, but are operating at a 2:1 rotational speed ratio, with arm A1 operating faster than arm A2. The arms A1, A2 will have the same displacement (x=y) when the ends of arms A1, A2 are coupled to the same holes 36 in flanges F5, F6. In this example, the resultant abrasion pattern 18 is a figure eight. This example utilizes a single motor M1 or M2 to operate belts BE1 and BE2, which in turn drives arms A1, A2 via connected flanges F3, F4.

Fig. 6 depicts an example device 10 where the arms A1, A2 have the same displacement (x=y), are in phase, and are operating at the same rotational speed ratio. The resultant abrasion pattern 18 is a straight line. As with the examples in Fig. 4, a single motor M1 or M2 drives belt BE1, which, in turn drives pulley P2. Pulley P2 is coupled to shaft S2 and drives shaft S4. Shaft S4 is coupled to shaft S2 by flanges F1, F2, which are fixed to one another in phase.

Fig. 7 depicts yet another example device 10 where the arms A1, A2 have the same displacement (x=y) and are operating at the same rotational speed ratio, but are out of phase 180 degrees. The resultant abrasion pattern 18 is an arc. The operation of this device is similar to that described above for Figs. 4 and 6, where a single motor M1 or M2 drives a single belt BE1. In this example, flanges F1, F2 are out of phase 180 degrees. Therefore, the details of operation will not be repeated.

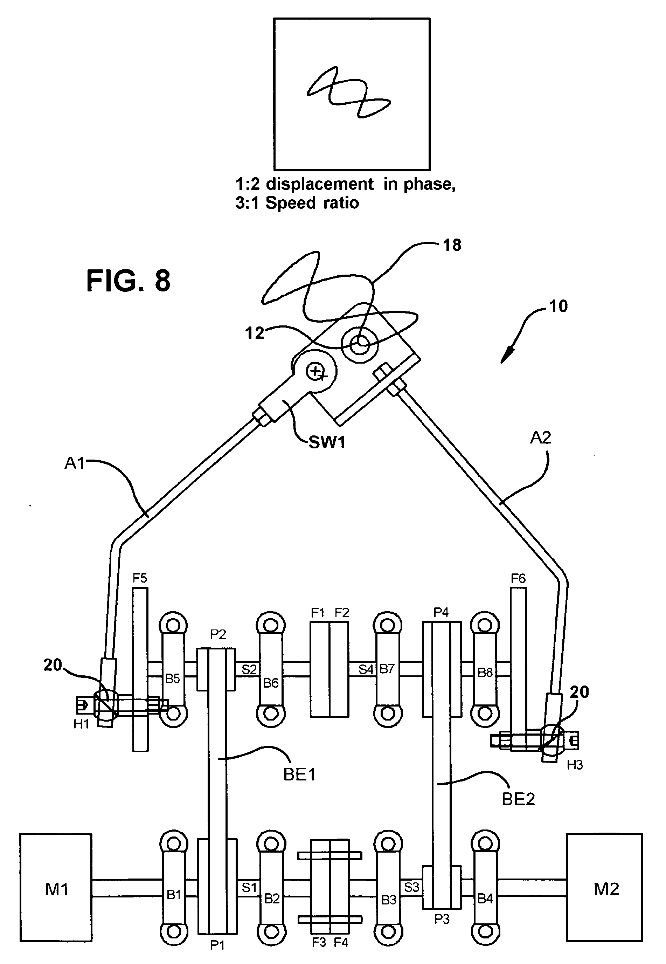

Fig. 8 depicts another example device 10 where the arms A1, A2 are displaced by a ratio of 1:2 (2x=y) and are in phase with one another. Once again, displacement of arms A1, A2 relative to one another is determined by which holes 36 are selected in flanges F5, F6. In this case, a hole 36 in flange F5 is chosen that is different from the hole chosen in flange F6 such that arm A1 is spaced a distance 2x while arm A2 is spaced a distance y. The rotational speed ratio is 3:1 between the first arm A1 and the second arm A2. The resultant abrasion pattern 18 is an extended figure eight, where three loops are created. In this example, the first or second motors M1, M2 can operate to drive belts BE1 and BE2 via coupled flanges F3 and F4.

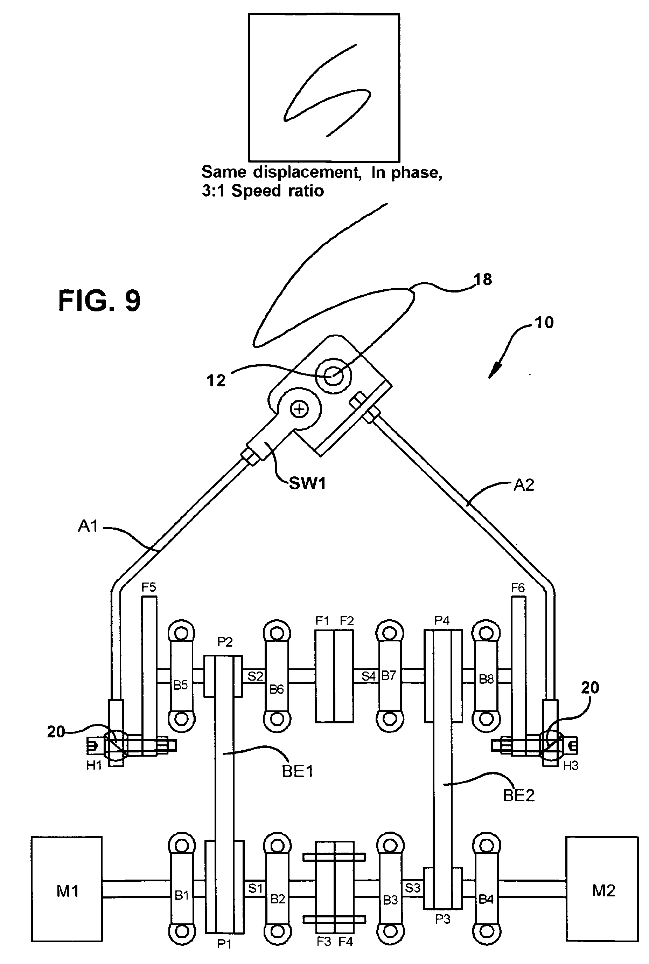

Fig. 9 depicts yet another example device 10 where the arms A1, A2 have the same displacement and are operating in phase with one another at a rotational speed ratio of 3:1. The resultant abrasion pattern 18 is an S-shaped pattern. The operation of this device is similar to that described above in connection with Fig. 8.

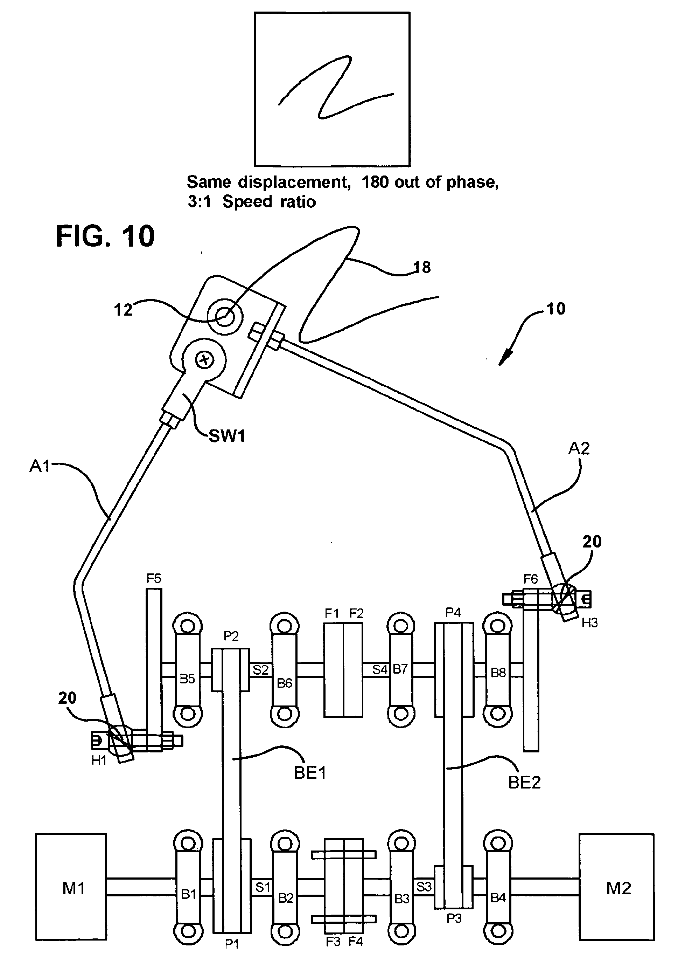

Fig. 10 depicts another example device 10 where the arms A1, A2 have the same displacement (x=y), are 180 degrees out of phase, and are operating at a rotational speed ratio of 3:1. The resulting abrasion pattern 18 is a Z-shape. The operation of this device is similar to that described above in connection with Fig. 8.

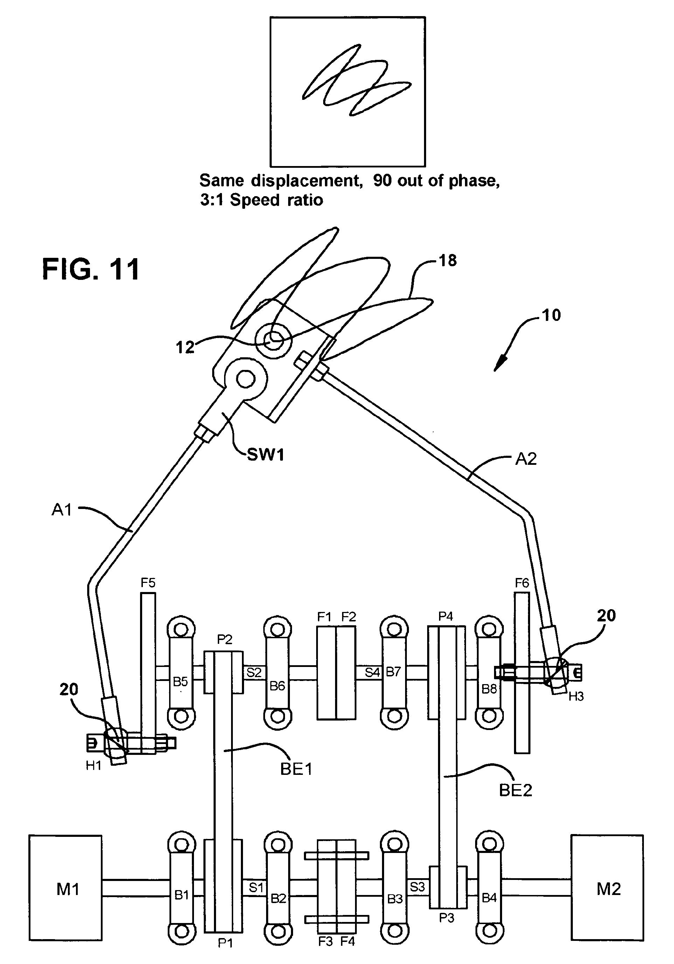

Fig. 11 depicts another example device 10 where the arms A1, A2 have the same displacement (x=y), are 90 degrees out of phase, and are operating at a rotational speed ratio of 3:1. The resulting abrasion pattern 18 is a modified figure eight pattern having three loops that is similar to that shown in Fig. 8, but wider in dimension than the pattern shown in Fig. 8. The operation of this device is similar to that described above in connection with Fig. 8.

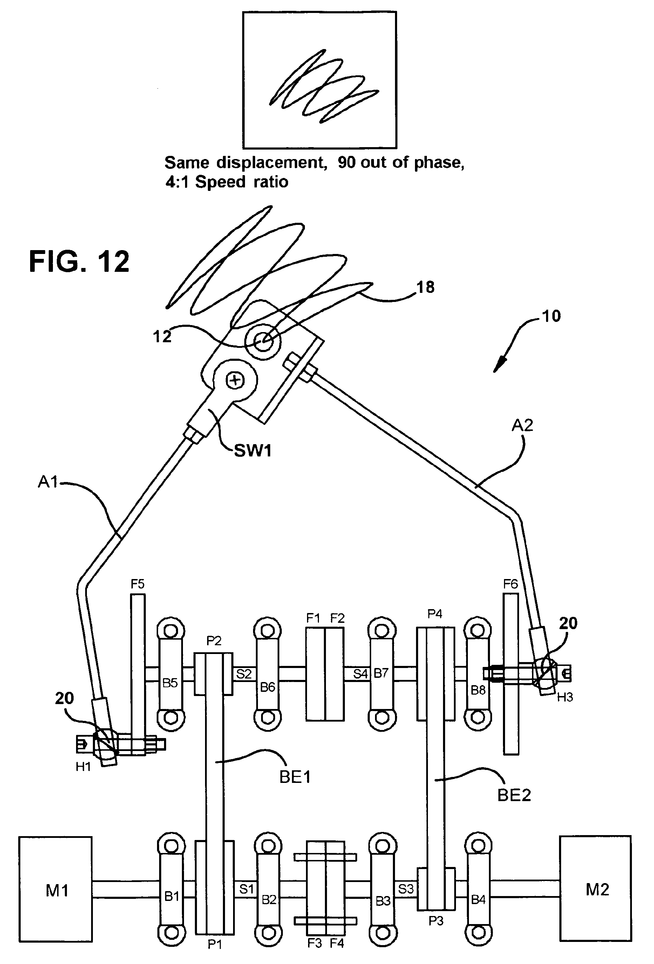

Fig. 12 depicts a further example device 10 where the arms A1, A2 have the same displacement (x=y), are 90 degrees out of phase with one another, and operate at a rotational speed ratio of 4:1. The resulting abrasion pattern 18 is a modified figure eight pattern having four loops that is similar to that shown in Fig. 11. The operation of this device is similar to that described above in connection with Fig. 8.

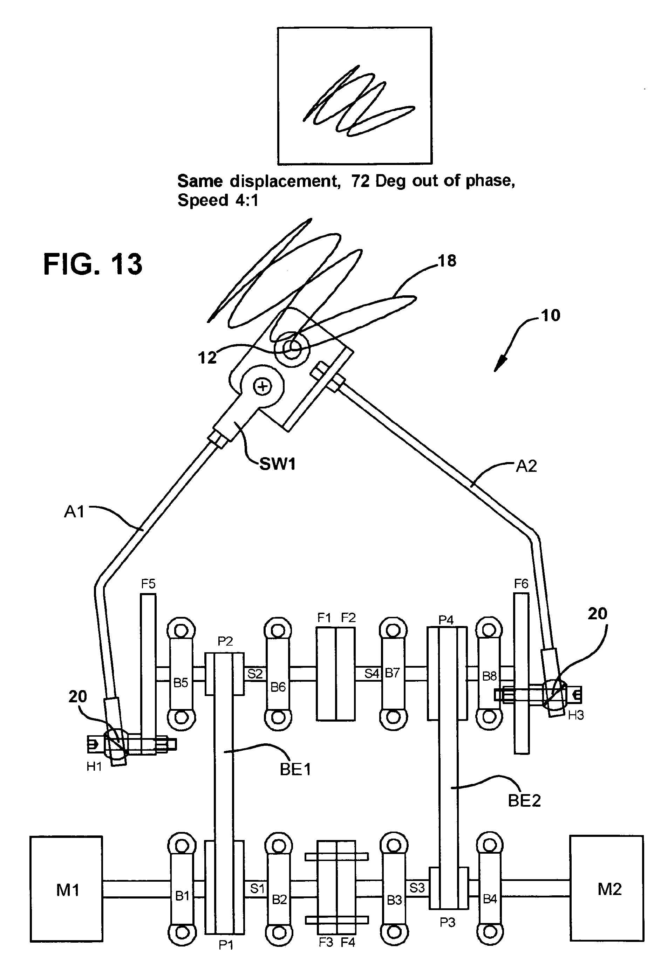

Fig. 13 depicts a further example device 10 where the arms A1, A2 have the same displacement (x=y), are 72 degrees out of phase with one another, and operate at a rotational speed ratio of 4:1. The resulting abrasion pattern 18 is a modified figure eight pattern having four loops that is similar to that shown in Fig. 12, but the loops are flatter than the loops in the pattern shown in Fig. 12. The operation of this device is similar to that described above in connection with Fig. 8.

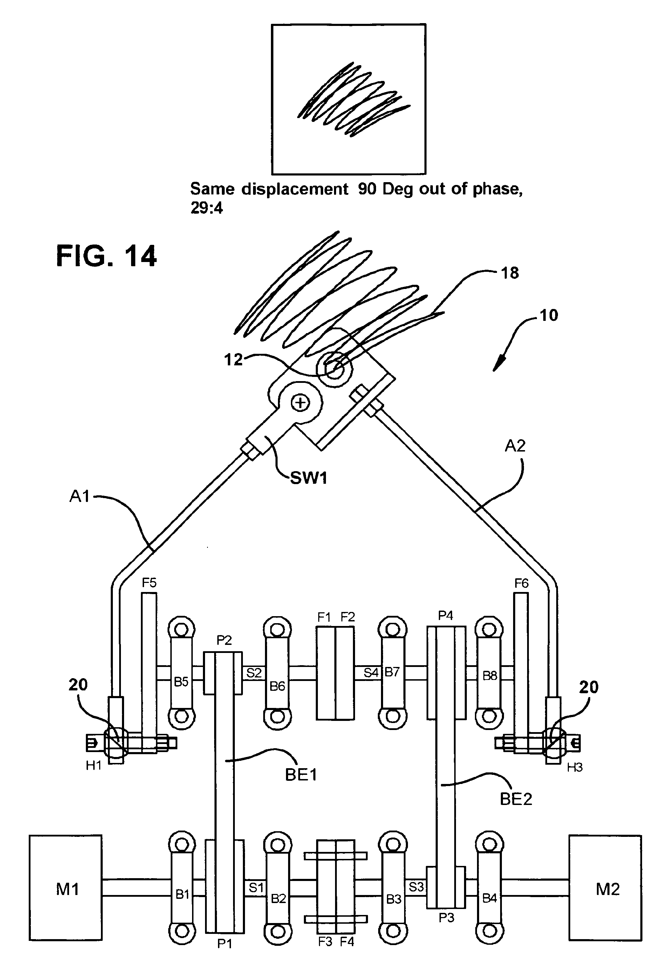

Fig. 14 depicts a further example device 10 where the arms A1, A2 have the same displacement (x=y), are 90 degrees out of phase with one another, and operate at a rotational speed ratio of 29:4. The resulting abrasion pattern 18 is a modified figure eight pattern having seven loops that is similar to that shown in Fig. 12, but with the loops being flatter than the loops depicted in Fig. 12. The operation of this device is similar to that described above in connection with Fig. 8.

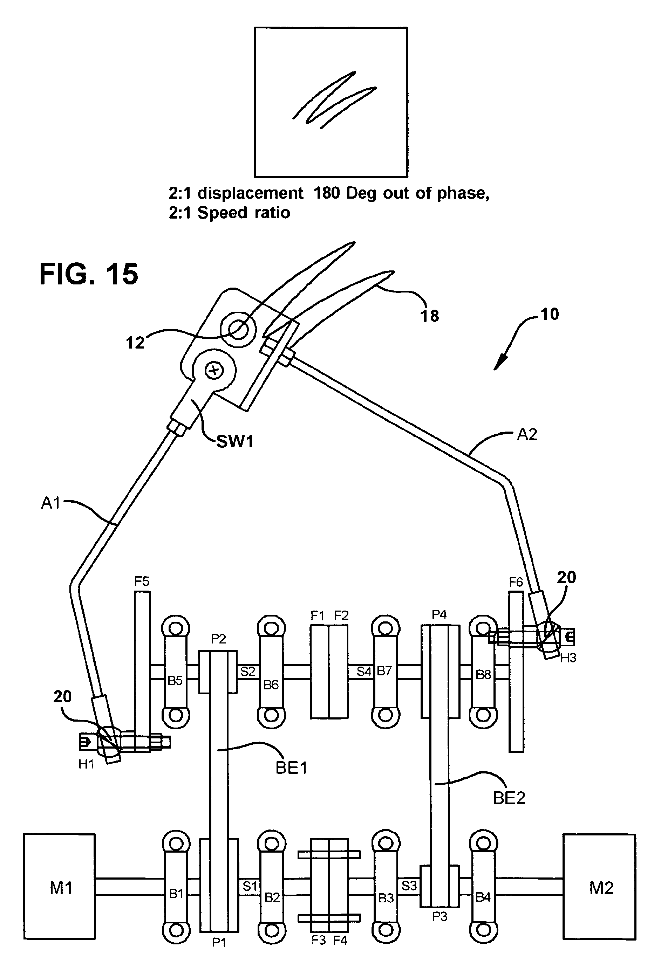

Fig. 15 depicts a further example device 10 where the arms A1, A2 have a 2:1 displacement ratio (x=2y), are 180 degrees out of phase with one another, and operate at a rotational speed ratio of 2:1. The resulting abrasion pattern 18 is an M-shaped pattern. The operation of this device is similar to that described above in connection with Fig. 8.

Fig. 16 depicts a front view of the example device 10 where the arms A1, A2 are out of phase with one another by 180 degrees and represents the resulting roll and slew of the frame T1 and abrasion tool 12. Figs. 19 and 20 depict a side view of the example shown in Fig. 9, with Fig. 19 representing a view of the right side and Fig. 20 representing a view of the left side of the device 10.

Figs. 17-18 show the lengths of alternative pointers or abrasion tools 12 which are different from the tools shown in Figs. 16 and 19-20. Fig. 17 is a side view of an example surface abrading device with a different abrasion tool. The device in Fig. 17 has a working plane that is above the axis of rotation of flange F6. Because of the orientation of the pointer on the working surface relative to the axis of rotation of the arm A2, when the pointer is pulled back toward shafts S2, S4, it pushes down harder than when the pointer is pushed away from shafts S2, S4. Fig. 18 is a side view of an example surface abrading device with a different abrasion tool holder. In this example, the working plane is below the axis of rotation of flange F5. This configuration causes the abrader tip 12 to push down harder onto the working plane when arm A2, and thus abrader tip 12, moves away from shafts S2 and S4, while the abrader tip does not push down as hard when the tip 12 is pulled toward shafts S2 and S4. As is evident, it is possible to change the force applied by the pointer 12 by changing the location of the working plane relative to the axis of rotation and by changing the length and configuration of the pointer 12.

Figs. 19 and 20 depict a side view of a device 10 where the arms A1, A2 have the same displacement, are in phase with one another, and are operating at a rotational speed ratio of 4:1. Fig. 19 represents the pitch and yaw of the frame T1 and tool holder 12. Fig. 20 shows the stroke of the device 10 and also depicts how the phase of the device can be adjusted by rotating flange F1 relative to flange F2, with flange F1 having pins 32 and flange F2 having holes 34 at several locations for receiving the pins 32 resulting in different phase differences between flanges F1, F2.

Figs. 1, 2, and 4-19 show different examples of the motion of the example surface abrader 10. The drawings demonstrate some of the motion capabilities of the example device, but do not show every possible abrading path of the pointer tip 12.

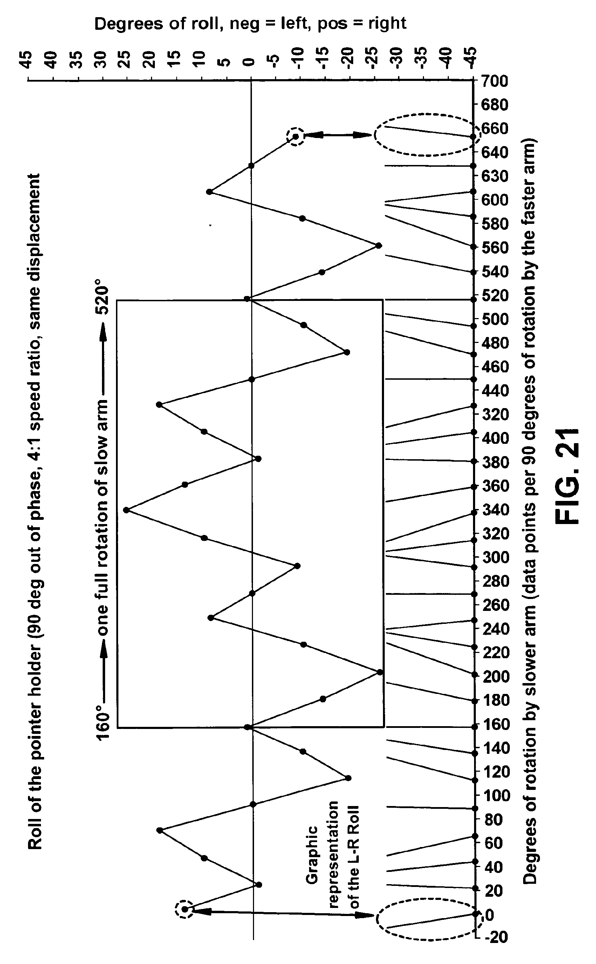

Fig. 21 is a diagram that represents the degrees of roll of the pointer 12 when the device is set up under the conditions of 90 degrees out of phase, a rotational speed ratio of 4:1, and the same arm displacement (x=y). This diagram is a graphical representation of the rolling motion of the pointer tip 12 at one specific setting of the abrader 10. The upper line shows the angle of roll of the pointer 12. The lower lines represent an approximate graphical representation of the roll at the same points. The lower lines are given as degrees of rotation of the slow arm for every 90 degrees rotation of the fast arm. The upper line represents more than one rotation of the slow arm. The boxed in area of the diagram represents one full rotation of the slow arm. Note also that a similar motion will be seen in the "yaw" and "pitch," but this has not been shown. The motion in the other directions will be of a similar style, but in a different pattern. Yaw, pitch, roll, and slew all occur simultaneously.

The scope of the invention is defined as set forth in the appended claims.

### List of Terms

- 10: surface abrader
- 12: abrasion tool
- 14: top end of frame
- 18: patterns
- 20: heim joint
- 22: ball swivel
- 24: opening
- 26: circular casing
- 28: threaded shaft
- 32: pins
- 34: ho les
- 36: arm offset holes

## Claims

1. An apparatus (10) for contacting a surface to be abraded or tested for surface abrasion comprising:
at least one motor (M1, M2);
a first shaft (S1, S3) coupled to the at least one motor (M1, M2);
a second shaft (S2, S4) coupled to the first shaft (S1, S3);
a first arm (A1) coupled via a first multi-axis joint (H1) to one end of the second shaft (S2, S4);
a second arm (A2) coupled via a second multi-axis joint (H2) to the other end of the second shaft (S2, S4); and **characterised by**
an abrasion tool (16) coupled between the first and second arms (A1, A2) for contacting a surface, wherein the first shaft (S1, S3), the second shaft (S2, S4), the first arm (A1), the second arm (A2), the first multi-axis joint (H1), the second multi-axis joint (H2) and the abrasion tool (16) work together to provide yaw, pitch, roll, stroke and movement of the abrasion tool (12).

2. The apparatus (10) of claim 1, wherein the first shaft (S1, S3) includes a first portion (S1) and a second portion (S3) and the second shaft (S2, S4) includes a first portion (S2) and a second portion (S4), with the first arm (A1) being coupled to the first portion (S2) of the second shaft (S2, S4) and the second arm (A2) being coupled to the second portion (S4) of the second shaft (S2, S4), with the first arm (A1) being movable either in phase or out of phase with respect to the second arm (A2).

3. The apparatus (10) of claim 2, wherein the at least one motor (M1, M2) includes a first motor (M1) for driving the first portion (S1) of the first shaft (S1, S3) and a second motor (M2) for driving the second portion (S3) of the first shaft (S1, S3).

4. The apparatus (10) of claim 2 or 3, wherein the first and second portions (S2, S4) of the second shaft (S2, S4) are coupled together such that they rotate at the same speed and different phases, or the same speed and the same phase.

5. The apparatus (10) of any of claims 2 to 4, wherein the first portion (S1) of the first shaft (S1, S3) is coupled to the second portion (S3) of the first shaft (S1, S3) via a first flange (F1) and a second flange (F2), and the first portion (S2) of the second shaft (S2, S4) is coupled to the second portion (S4) of the second shaft (S2, S4) via a third and a fourth flange (F3, F4), and the first portion (S2) of the second shaft (S2, S4) is coupled to the first arm (A1) via a fifth flange (F5) and the second portion (S4) of the second shaft (S2, S4) is coupled to the second arm (A2) via a sixth flange (F6), and the first and second flanges (F1, F2) may be one of fixed to each other or movable relative to each other, and the third and fourth flanges (F3, F4) may be one of fixed to each other or movable relative to each other, wherein the first and second flanges (F1, F2), when coupled together may determine the phase of the first arm (A1) relative to the second arm (A2) and the third and fourth flanges (F3, F4), when coupled together, may determine the phase of the first arm (A1) relative to the second arm (A2).

6. The apparatus (10) of claim 5, wherein the first flange (F1) is fixedly coupled to the second flange (F2) and the third flange (F3) is movable relative to the fourth flange (F4), and the first flange (F1) has at least one hole (32) extending therethrough and the second flange (F2) has a plurality of holes (34) extending therethrough around the periphery thereof, and a pin extends through the at least one hole (32) in the first flange (F1) into one of said plurality of holes (34) in the second flange (F2) in order to select a phase shift from 0 to 360 degrees for the first arm (A1) relative to the second arm (A2).

7. The apparatus (10) according to one of claims 1-6, **characterized by** further comprising:
a plurality of pulleys (P1, P2, P3, P4), with at least some of the pulleys being coupled to the first motor (M1, M2);
a plurality of shafts (S1, S2, S3, S4), with the pulleys (P1, P2, P3, P4) being coupled to the shafts;
at least one belt (BE1, BE2) coupled between two of the pulleys (P1, P2, P3, P4) to drive one of said shafts (S1, S2, S3, S4) via rotation of the other shaft by the first motor (M1, M2),
and said arms (A1, A2) moving in a reciprocating motion.

8. The apparatus (10) of claim 7, wherein the reciprocating motion produces an abrasion pattern (18) and the pattern (18) is either a line pattern or an area pattern.

9. The apparatus (10) of claim 7 or 8, further comprising a frame (T1) coupled between the first and second arms (A1, A2) via the multi-axis joints (H1, H2, H3), with the abrasion tool (12) being coupled to the frame (T1).

10. The apparatus (10) of claim 9, further comprising a second motor (M1, M2) coupled to at least some of the pulleys (P1, P2, P3, P4) and the plurality of shafts (S1, S2, S3, S4) comprising a first shaft (S1, S3) and a second shaft (S2, S4), with the first shaft (S1, S3) being displaced from, but parallel to the second shaft (S2, S4), said second shaft (S2, S4) having the at least one movable joint (H1, H2, H3) associated with both ends thereof, said joints (H1, H2, H3) each being coupled to the first and second arms (A1, A2).

11. The apparatus (10) of any of claims 7 to 10, wherein the abrasion tool (12) is a pointer, or a finger-like pointer, or an abrasion device.

12. The apparatus (10) of claim 11, further comprising a plurality of bearings (B1, B2, B3, B4, B5, B6, B7, B8) interspersed between the motors (M1, M2) and pulleys (P1, P2, P3, P4).

13. The apparatus (10) of any of claims 7 to 12, wherein the plurality of shafts (S1, S2, S3, S4) comprises a first shaft (S1, S3) and a second shaft (S2, S4), with at least one pulley (P1, P2, P3, P4) coupled to each of the shafts (S1, S2, S3, S4) and with opposing pulleys (P1, P2, P3, P4) coupled to one another via at least one belt (BE1, BE2).

14. The apparatus (10) of claim 13, wherein the first shaft (S1, S3) comprises a first portion (S1) and a second portion (S3), with the first portion (S1) being coupled to the second portion (S3) via at least one flange (F3, F4), and the second shaft (S2, S4) comprises a first portion (S2) and a second portion (S4), with the first portion (S2) being coupled to the second portion (S4) via at least one flange (F1, F2).

15. The apparatus (10) of claim 14, wherein the first arm (A1) is coupled to the first portion (S2) of the second shaft (S2, S4) and the second arm (A2) is coupled to the second portion (S4) of the second shaft (S2, S4), the at least one flange (F2, F2) that couples the first and second portions (S2, S4) of the second shaft includes first and second flanges (F1, F2) that are coupled by at least one pin, with at least one of the first or second flanges (F1, F2) having a plurality of openings (32, 34) spaced around a face thereof for accepting the at least one pin therein, said at least one pin and openings (32, 34) for adjusting the phase of the first arm (A1) relative to the second arm (A2).

16. The apparatus (10) of claim 14 or 15, further comprising a fifth flange (F5) coupled between the first portion (S2) of the second shaft (S2, S4) and the first arm (A1), with a first movable joint (H1) coupled between the fifth flange (F5) and the first arm (A1); a sixth flange (F6) coupled between the second portion (S4) of the second shaft (S2, S4) and the second arm (A2), with a second movable joint (H3) coupled between the sixth flange (F6) and the second arm (A2); and a third movable joint (H3) coupled between the first arm (A1) and the abrasion tool (16).

## Patentansprüche

1. Vorrichtung (10) zum Kontaktieren einer Oberfläche, die abgenutzt bzw.
verschlissen oder auf eine Oberflächenabnutzung getestet werden soll, die aufweist:
zumindest einen Motor (M1, M2);
eine erste Welle (S1, S3), die mit dem zumindest einen Motor (M1, M2) verbunden ist;
eine zweite Welle (S2, S4), die mit der ersten Welle (S1, S3) verbunden ist;
einen ersten Arm (A1), der über eine erste Multi-Achsen-Verbindung (H1) mit einem Ende der zweiten Welle (S2, S4) verbunden ist;
einen zweiten Arm (A2), der über eine zweite Multi-Achsen-Verbindung (H2) mit dem anderen Ende der zweiten Welle (S2, S4) verbunden ist; und
**gekennzeichnet durch**
ein Abnutzungsgerät (16), das zwischen den ersten und zweiten Armen (A1, A2) gekoppelt ist zum Kontaktieren einer Oberfläche, wobei die erste Welle (S1, S3), die zweite Welle (S2, S4), der erste Arm (A1), der zweite Arm (A2),
die erste Muiti-Achsen-Verbindung (H1), die zweite Multi-Achsen-Verbindung (H2) und das Abnutzungsgerät (16) zusammenarbeiten, um Gierung, Neigung, Rollen, Takt und Bewegung des Abnutzungsgeräts (12) vorzusehen.

2. Vorrichtung (10) gemäß Anspruch 1, wobei die erste Welle (S1, S3) einen ersten Abschnitt (S1) und einen zweiten Abschnitt (S3) umfasst und die zweite Welle (S2, S4) einen ersten Abschnitt (S2) und einen zweiten Abschnitt (S4) umfasst, wobei der erste Arm (A1) mit dem ersten Abschnitt (S2) der zweiten Welle (S2, S4) verbunden ist und der zweite Arm (A2) mit dem zweiten Abschnitt (S4) der zweiten Welle (S2, S4) verbunden ist, wobei der erste Arm (A1) entweder in Phase oder phasenverschoben in Bezug auf den zweiten Arm (A2) bewegt werden kann.

3. Vorrichtung (10) gemäß Anspruch 2, wobei der zumindest eine Motor (M1, M2) einen ersten Motor (M1) zum Antreiben des ersten Abschnitts (S1) der ersten Welle (S1, S3) und einen zweiten Motor (M2) zum Antreiben des zweiten Abschnitts (S3) der ersten Welle (S1, S3) umfasst.

4. Vorrichtung (10) gemäß Anspruch 2 oder 3, wobei die ersten und zweiten Abschnitte (S2, S4) der zweiten Welle (S2, S4) derart miteinander verbunden sind, dass sie mit der gleichen Geschwindigkeit und unterschiedlichen Phasen oder mit der gleichen Geschwindigkeit und der gleichen Phase rotieren.

5. Vorrichtung (10) gemäß einem der Ansprüche 2 bis 4, wobei der erste Abschnitt (S1) der ersten Welle (S1, S3) mit dem zweiten Abschnitt (S3) der ersten Welle (S1, S3) über einen ersten Flansch (F2) und einen zweiten Flansch (F2) verbunden ist, und der erste Abschnitt (S2) der zweiten Welle (S2, S4) mit dem zweiten Abschnitt (S4) der zweiten Welle (S2, S4) über einen dritten und vierten Flansch (F3, F4) verbunden ist, und der erste Abschnitt (S2) der zweiten Welle (S2, S4) mit dem ersten Arm (A1) über einen fünften Flansch (F5) verbunden ist und der zweite Abschnitt (S4) der zweiten Welle (S2, S4) mit dem zweiten Arm (A2) über einen sechsten Flansch (F6] verbunden ist, und die ersten und zweiten Flansche (F1, F2) eines sein können aus fest zueinander oder beweglich relativ zueinander, und die dritten und vierten Flansche (F3, F4) eines sein können aus fest zueinander oder beweglich relativ zueinander, wobei die ersten und zweiten Flansche (F1, F2), wenn miteinander verbunden, die Phase des ersten Arms (A1) relativ zu dem zweiten Arm (A2) bestimmen können, und die dritten und vierten Flansche (F3, F4), wenn miteinander verbunden, die Phase des ersten Arms (A1) relativ zu dem zweiten Arm (A2) bestimmen können.

6. Vorrichtung (10) gemäß Anspruch 5, wobei der erste Flansch (F1) fest mit dem zweiten Flansch (F2) verbunden ist und der dritte Flansch (F3) beweglich ist relativ zu dem vierten Flansch (F4), und der erste Flansch (F1) zumindest ein sich **dadurch** erstreckendes Loch (32) hat und der zweite Flansch (F2) eine Vielzahl von sich **dadurch** erstreckenden Löchern (34) hat um dessen Umfang, und wobei sich ein Stift durch das zumindest eine Loch (32) in dem ersten Flansch (F1) in eines der Vielzahl von Löchern (34) in dem zweiten Flansch (F2) erstreckt, um eine Phasenverschiebung von O bis 360 Grad für den ersten Arm (A1) relativ zu dem zweiten Arm (A2) auszuwählen.

7. Vorrichtung (10) gemäß einem der Ansprüche 1-6, **dadurch gekennzeichnet, dass** sie weiter aufweist:
eine Vielzahl von Riemenscheiben (P1, P2, P3, P4), wobei zumindest einige der Riemenscheiben mit dem ersten Motor (M1, M2) verbunden sind;
eine Vielzahl von Wellen (S1, S2, S3, S4), wobei die Riemenscheiben (P1, P2, P3, P4) mit den Wellen verbunden sind;
zumindest ein Riemen (BE1, BE2), der zwischen zwei der Riemenscheiben (P1, P2, P3, P4) verbunden ist, um eine der Wellen (S1, S2, S3, S4) über eine Rotation der anderen Welle durch den ersten Motor (M1, M2) anzutreiben,
und wobei sich die Arme (A1, A2) in einer hin- und hergehenden Bewegung bewegen.

8. Vorrichtung (10) gemäß Anspruch 7, wobei die hin- und hergehende Bewegung ein Abnutzungsmuster (18) erzeugt und das Muster (18) entweder ein Linienmuster oder ein Flächenmuster ist.

9. Vorrichtung (10) gemäß Anspruch 7 oder 8, die weiter einen Rahmen (T1) aufweist, der zwischen den ersten und zweiten Armen (A1, A2) über die Multi-Achsen Verbindungen (H1, H2, H3) verbunden ist, wobei das Abnutzungsgerät (12) mit dem Rahmen (T1) verbunden ist.

10. Vorrichtung (10) gemäß Anspruch 9, die weiter aufweist einen zweiten Motor (M1, M2), der mit zumindest einigen der Riemenscheiben (P1, P2, P3, P4) und der Vielzahl von Wellen (S1, S2, S3, S4) verbunden ist, die eine erste Welle (S1, S3) und eine zweite Welle (S2, S4) aufweisen, wobei die erste Welle (S1, S3) versetzt, aber parallel ist zu der zweiten Welle (S2, S4), wobei die zweite Welle (S2, S4) die zumindest eine bewegliche Verbindung (H1, H2, H3) hat, die zu deren beiden Enden gehört, wobei die Verbindungen (H1, H2, H3) jeweils mit den ersten und zweiten Armen (A1, A2) verbunden sind.

11. Vorrichtung (10) gemäß einem der Ansprüche 7 bis 10, wobei das Abnutzungsgerät (12) ein Zeiger oder ein Finger-ähnlicher Zeiger oder eine Abnutzungsvorrichtung ist.

12. Vorrichtung (10) gemäß Anspruch 11, die weiter eine Vielzahl von Lagern (B1, B2, B3, B4, B5, B6, B7, B8) aufweist, die zwischen den Motoren (M1, M2) und Riemenscheiben (P1, P2, P3, P4) eingefügt sind.

13. Vorrichtung (10) gemäß einem der Ansprüche 7 bis 12, wobei die Vielzahl von Wellen (S1, S2, S3, S4) eine erste Welle (S1, S3) und eine zweite Welle (S2, S4) aufweisen, wobei zumindest eine Riemenscheibe (P1, P2, P3, P4) mit jeder der Wellen (S1, S2, S3, S4) verbunden ist und wobei gegenüberliegende Riemenscheiben (P1, P2, P3, P4) über zumindest einen Riemen (BE1, BE2) miteinander verbunden sind.

14. Vorrichtung (10) gemäß Anspruch 13, wobei die erste Welle (S1, S3) einen ersten Abschnitt (S1) und einen zweiten Abschnitt (S3) aufweist, wobei der erste Abschnitt (S1) mit dem zweiten Abschnitt (S3) über zumindest einen Flansch (F3, F4) verbunden ist, und die zweite Welle (S2, S4) einen ersten Abschnitt (S2) und einen zweiten Abschnitt (S4) aufweist, wobei der erste Abschnitt (S2) mit dem zweiten Abschnitt (S4) über zumindest einen Flansch (F1, F2) verbunden ist.

15. Vorrichtung (10) gemäß Anspruch 14, wobei der erste Arm (A1) mit dem ersten Abschnitt (S2) der zweiten Welle (S2, S4) verbunden ist und der zweite Arm (A2) mit dem zweiten Abschnitt (S4) der zweiten Welle (S2, S4) verbunden ist, wobei der zumindest eine Flansch (F1, F2), der die ersten und zweiten Abschnitte (S2, 54) der zweiten Welle verbindet, erste und zweite Flansche (F1, F2) umfasst, die durch zumindest einen Stift verbunden sind, wobei zumindest einer der ersten oder zweiten Flansche (F1, F2) eine Vielzahl von Öffnungen (32, 34) hat, die um eine Fläche davon mit Abstand angeordnet sind, zum darin Aufnehmen des zumindest einen Stifts, wobei der zumindest eine Stift und die Öffnungen (32, 34) vorgesehen sind zum Anpassen der Phase des ersten Arms (A1) relativ zu dem zweiten Arm (A2).

16. Vorrichtung (10) gemäß Anspruch 14 oder 15, die weiter aufweist einen fünften Flansch (F5), der zwischen dem ersten Abschnitt (S2) der zweiten Welle (S2, S4) und dem ersten Arm (A1) verbunden ist, wobei eine erste bewegliche Verbindung (H1) zwischen dem fünften Flansch (F5) und dem ersten Arm (A1) verbunden ist; einen sechsten Flansch (F6), der zwischen dem zweiten Abschnitt (S4) der zweiten Welle (S2, S4) und dem zweiten Arm (A2) verbunden ist, wobei eine zweite bewegliche Verbindung (H3) zwischen dem sechsten Flansch (F6) und dem zweiten Arm (A2) verbunden ist; und eine dritte bewegliche Verbindung (H3) zwischen dem ersten Arm (A1) und dem Abnutzungsgerät (16) verbunden ist.

## Revendications

1. Appareil (10) destiné à venir au contact d'une surface à abraser ou à soumettre à un test d'abrasion de surface, comprenant :
au moins un moteur (M1, M2) ;
un premier arbre (S1, S3) relié à l'au moins un moteur (M1, M2) ;
un deuxième arbre (S2, S4) relié au premier arbre (S1, S3) ;
un premier bras (A1 relié par l'intermédiaire d'une première articulation multiaxiale (H1 à une extrémité du deuxième arbre (S2, S4) ;
un deuxième bras (A2) relié par l'intermédiaire d'une deuxième articulation multiaxiale (H2) à l'autre extrémité du deuxième arbre (S2, S4) ; et **caractérisé par** :
un outil d'abrasion (16) relié entre les premier et deuxième bras (A1 A2) destiné à venir au contact d'une surface, le premier arbre (S1, S3), le deuxième arbre (S2, S4), le premier bras (A1, le deuxième bras (A2), la première articulation multiaxiale (H1, la deuxième articulation multiaxiale (H2) et l'outil d'abrasion (16) fonctionnant ensemble pour conférer à l'outil d'abrasion (12) un mouvement de lacet, de tangage, de roulis, de course et de déplacement.

2. Appareil (10) selon la revendication 1, dans lequel le premier arbre (S1, S3) comporte une première partie (S1 et une deuxième partie (S3) et le deuxième arbre (S2, S4) comporte une première partie (S2) et une deuxième partie (S4), le premier bras (A1) étant relié à la première partie (S2) du deuxième arbre (S2, S4) et le deuxième arbre (A2) étant relié à la deuxième partie (S4) du deuxième arbre (S2, S4), le premier bras (A1 étant mobile soit en phase, soit de manière déphasée par rapport au deuxième bras (A2).

3. Appareil (10) selon la revendication 2, dans lequel l'au moins un moteur (M1 M2) comporte un premier moteur (M1 destiné à entraîner la première partie (S1 du premier arbre (S1, S3) et un deuxième moteur (M2) destiné à entraîner la deuxième partie (S3) du premier arbre (S1, S3).

4. Appareil (10) selon la revendication 2 ou 3, dans lequel les première et deuxième parties (S2, S4) du deuxième arbre (S2, S4) sont reliées l'une à l'autre de façon à ce qu'elles tournent à la même vitesse et avec des phases différentes, ou à la même vitesse et avec la même phase.

5. Appareil (10) selon l'une quelconque des revendications 2 à 4, dans lequel la première partie (S1 du premier arbre (S1, S3) est reliée à la deuxième partie (S3) du premier arbre (S1, S3) par l'intermédiaire d'une première flasque (F1 et d'une deuxième flasque (F2), et la première partie (S2) du deuxième arbre (S2, S4) est reliée à la deuxième partie (S4) du deuxième arbre (S2, S4) par l'intermédiaire d'une troisième et d'une quatrième flasques (F3, F4), et la première partie (S2) du deuxième arbre (S2, S4) est reliée au premier bras (A1 par l'intermédiaire d'une cinquième flasque (F5) et la deuxième partie (S4) du deuxième arbre (S2, S4) est reliée au deuxième arbre (A2) par l'intermédiaire d'une sixième flasque (F6), et les première et deuxième flasques (F1 F2) peuvent être soit fixées l'une à l'autre, soit mobiles l'une par rapport à l'autre, et les troisième et quatrième flasques (F3, F4) peuvent être soit fixées l'une à l'autre, soit mobiles l'une par rapport à l'autre, dans lequel les première et deuxième flasques (F1, F2), lorsqu'elles sont reliées l'une à l'autre, peuvent déterminer la phase du premier bras (A1) par rapport au deuxième bras (A2) et les troisième et quatrième flasques (F3, F4), lorsqu'elles sont reliées l'une à l'autre, peuvent déterminer la phase du premier bras (A1) par rapport au deuxième bras (A2).

6. Appareil (10) selon la revendication 5, dans lequel la première flasque (F1) est reliée de façon fixe à la deuxième flasque (F2) et la troisième flasque (F3) est mobile par rapport à la quatrième flasque (F4), et la première flasque (F1) présente au moins un trou (32) la traversant et la deuxième flasque (F2) présente une pluralité de trous (34) la traversant et le long de sa périphérie, et une broche s'étend dans l'au moins un trou (32) ménagé dans la première flasque (F1) en pénétrant dans l'un de ladite pluralité de trous (34) ménagés dans la deuxième flasque (F2) afin de sélectionner un déphasage de 0 à 360 degrés pour le premier bras (A1) par rapport au deuxième bras (A2).

7. Appareil (10) selon l'une des revendications 1 à 6, **caractérisé en ce qu'**il comprend en outre :
une pluralité de poulies (P1, P2, P3, P4), au moins certaines des poulies étant reliées au premier moteur (M1, M2) ;
une pluralité d'arbres (S1, S2, S3, S4), les poulies (P1, P2, P3, P4) étant reliées aux arbres ;
au moins une courroie (BE1, BE2) reliée entre deux des poulies (P1, P2, P3, P4) pour entraîner l'un desdits arbres (S1, S2, S3, S4) par mise en rotation de l'autre arbre par le premier moteur (M1, M2),
et lesdits bras (A1, A2) se déplaçant selon un mouvement de va-et-vient.

8. Appareil (10) selon la revendication 7, dans lequel le mouvement de va-et-vient produit un motif d'abrasion (18) et le motif (18) est soit un motif linéaire, soit un motif bidimensionnel.

9. Appareil (10) selon la revendication 7 ou 8, comprenant en outre un châssis (T1) relié entre les premier et deuxième bras (A1, A2) par l'intermédiaire des articulations multiaxiales (H1, H2, H3), l'outil d'abrasion (12) étant relié au châssis (T1).

10. Appareil (10) selon la revendication 9, comprenant en outre un deuxième moteur (M1, M2) relié à au moins certaines des poulies (P1, P2, P3, P4) et la pluralité d'arbres (S1, S2, S3, S4) comprenant un premier arbre (S1, S3) et un deuxième arbre (S2, S4), le premier arbre (S1, S3) étant déplacé par rapport au deuxième arbre (S2, S4), mais parallèlement à celui-ci, ledit deuxième arbre (S2, S4) ayant l'au moins une articulation mobile (H1, H2, H3) associée à ses deux extrémités, chacune desdites articulations (H1, H2, H3) étant reliée aux premier et deuxième bras (A1, A2).

11. Appareil (10) selon l'une quelconque des revendications 7 à 10, dans lequel l'outil d'abrasion (12) est une aiguille, ou une aiguille de type doigt, ou un dispositif d'abrasion.

12. Appareil (10) selon la revendication 11, comprenant en outre une pluralité de roulements (B1, B2, B3, B4, B5, B6, B7, B8) répartis entre les moteurs (M1, M2) et les poulies (P1, P2, P3, P4).

13. Appareil (10) selon l'une quelconque des revendications 7 à 12, dans lequel la pluralité d'arbres (S1, S2, S3, S4) comprend un premier arbre (S1, S3) et un deuxième arbre (S2, S4), au moins une poulie (P1, P2, P3, P4) étant reliée à chacun des arbres (S1, S2, S3, S4) et des poulies opposées (P1, P2, P3, P4) étant reliées les unes aux autres par l'intermédiaire d'au moins une courroie (BE1, BE2).

14. Appareil (10) selon la revendication 13, dans lequel le premier arbre (S1, S3) comprend une première partie (S1) et une deuxième partie (S3), la première partie (S1) étant reliée à la deuxième partie (S3) par l'intermédiaire d'au moins une flasque (F3, F4), et le deuxième arbre (S2, S4) comprend une première partie (S2) et une deuxième partie (S4), la première partie (S2) étant reliée à la deuxième partie (S4) par l'intermédiaire d'au moins une flasque (F1, F2).

15. Appareil (10) selon la revendication 14, dans lequel le premier bras (A1) est relié à la première partie (S2) du deuxième arbre (S2, S4) et le deuxième bras (A2) est relié à la deuxième partie (S4) du deuxième arbre (S2, S4), l'au moins une flasque (F1, F2) qui relie les première et deuxième parties (S2, S4) du deuxième arbre comporte des première et deuxième flasques (F1, F2) qui sont reliées par au moins une broche, au moins l'une des première et deuxième flasques (F1, F2) ayant une pluralité d'orifices (32, 34) espacés sur le pourtour d'une face de celle-ci pour accepter l'au moins une broche dans celle-ci, ladite au moins une broche et lesdits orifices (32, 34) étant destinés à ajuster la phase du premier bras (A1) par rapport au deuxième bras (A2).

16. Appareil (10) selon la revendication 14 ou 15, comprenant en outre une cinquième flasque (F5) reliée entre la première partie (S2) du deuxième arbre (S2, S4) et le premier bras (A1), une première articulation mobile (H1) étant reliée entre la cinquième flasque (F5) et le premier bras (A1) ; une sixième flasque (F6) reliée entre la deuxième partie (S4) du deuxième arbre (S2, S4) et le deuxième bras (A2), une deuxième articulation mobile (H3) étant reliée entre la sixième flasque (F6) et le deuxième bras (A2) ; et une troisième articulation mobile (H3) reliée entre le premier bras (A1) et l'outil d'abrasion (16).
